Europäisches Patentamt

European Patent Office

Office européen des brevets

(19)

(11) Veröffentlichungsnummer: **0 387 545**

**A1**

(12) **EUROPÄISCHE PATENTANMELDUNG**

(21) Anmeldenummer: **90103084.1**

(22) Anmeldetag: **17.02.90**

(51) Int. Cl.⁵: **C12N 1/06**

(30) Priorität: **17.03.89 DE 3908737**

(43) Veröffentlichungstag der Anmeldung:
**19.09.90 Patentblatt 90/38**

(84) Benannte Vertragsstaaten:
**AT BE CH DE ES FR GB GR IT LI LU NL SE**

(71) Anmelder: **MESSER GRIESHEIM GMBH**
**Hanauer Landstrasse 330**
**D-6000 Frankfurt/Main(DE)**

(72) Erfinder: **Buchmüller, Jürgen**
**Horstdyk 47**
**D-4150 Krefeld(DE)**
Erfinder: **Weyermanns, Günther**
**Hans Sachs Strasse 18**
**D-5142 Hückelhoven 6(DE)**
Erfinder: **Zerwas, Stefan**
**Rieselhof 8**
**D-4300 Essen(DE)**

(54) **Verfahren zum Aufschluss der Zellen von Mikroorganismen.**

(57) Zum Aufschluß der Zellen von Mikroorganismen zwecks Gewinnung der Zellinhaltsstoffe werden Konzentrate der Mikroorganismen mittels tiefsiedender verflüssigter Gase tiefgefroren und anschließend mechanisch aufgeschlossen.

EP 0 387 545 A1

## Verfahren zum Aufschluß der Zellen von Mikroorganismen

Die Erfindung betrifft ein Verfahren zum Aufschluß der Zellen von Mikroorganismen zwecks Gewinnung der Zellinhaltsstoffe.

Zur Gewinnung von Zellinhaltsstoffen aus Mikroorganismen, beispielsweise intrazellulärer, biologisch aktiver Proteine, ist die Zerstörung der Zellwand der Mikroorganismen der erste Verfahrensschritt. Die Zerstörung der Zellwände erfolgt mechanisch durch Naßmahlung von Konzentraten der Mikroorganismen in Kugelmühlen oder durch Hochdruckhomogenisation, bei welcher die Konzentrate mit großem Druck durch enge Spalten gepreßt werden. Daneben sind chemische und biologische Aufschlußverfahren bekannt. Unter Konzentrate sind hierbei Pufferlösungen mit darin befindlichen Mikroorganismen zu verstehen, beispielsweise wässrige Suspensionen.

Als Beispiel für den Aufschluß von Mikroorganismen sei Backhefe genannt. Aus ihr werden als Zellinhaltsstoffe Glukosidasen und Hexokinasen gewonnen. Diese werden in der Zucker-, Pharma- und Chemie-Industrie benötigt. Beispielsweise kann mit Hilfe von Amyloglukosidasen Glukose in Glukonsäure umgewandelt werden, die als Zusatzstoff für die Haltbarkeitsverlängerung von Erfrischungsgetränken, die Entfernung von Glukose aus Eiweißen oder für die Herstellung von kristallinen Traubenzucker benötigt wird. Hexokinasen werden in der Pharma-Industrie für die Übertragung von Molekülgruppen eingesetzt.

Der Erfindung liegt die Aufgabe zugrunde, ein Aufschlußverfahren für die Zellen von Mikroorganismen zu schaffen, welches einen besonders rationellen Betriebsablauf ermöglicht.

Ausgehend von dem im Oberbegriff des Anspruches 1 berücksichtigten Stand der Technik ist diese Aufgabe erfindungsgemäß gelöst mit den im kennzeichnenden Teil des Anspruches 1 angegebenen Merkmalen.

Vorteilhafte Weiterbildungen der Erfindung sind in den Unteransprüchen angegeben.

Bei dem erfindungsgemäßen Verfahren werden demnach wie bei den bekannten mechanischen Aufschlußverfahren zunächst Konzentrate der Mikroorganismen gebildet. Diese Konzentrate werden jedoch mit Hilfe von tiefsiedenden verflüssigten Gasen, beispielsweise flüssigem Stickstoff, tiefgefroren. Das Konzentrat kann je nach Art des Mikroorganismus langsam in Form einer Platte eingefroren werden oder schnell durch Eintropfen in das tiefsiedende verflüssigte Gas zu Pellets tiefgefroren werden. Dadurch wird ein innerer Aufschluß (teilweise Aufschluß der Zellmembrane erreicht. Das Vorzerkleinern der gefrorenen Platten und das anschließende Kaltvermahlen, beispielsweise in Stiftmühlen (Korngrößen von 100 μm) oder Gasstrahlmühlen (Korngrößen zwischen 5 und 10 μm), ermöglicht das Freilegen der Zellinhaltsstoffe (vollständiger Aufschluß). Als tiefsiedendes verflüssigtes Gas kommt in erster Linie Stickstoff wegen seiner leichten Verfügbarkeit in Betracht, das erfindungsgemäße Verfahren könnte aber auch in Sonderfällen mit flüssigem Kohlendioxid durchgeführt werden.

Ein besonderer Vorteil des erfindungsgemäßen Verfahrens besteht darin, daß die tiefgefrorenen Konzentrate über lange Zeiträume gelagert werden können, bevor sie gemahlen werden. Die Verfahrensschritte der Konzentrateinfrierung und der Mahlung können daher völlig unabhängig voneinander stattfinden. und jeder für sich auf rationelle Weise durchgeführt werden. Die bekannten mechanischen Aufschlußverfahren bieten diese Möglichkeit nicht. Das erfindungsgemäße Verfahren ist darüber hinaus besonders für solche Mikroorganismen besonders geeignet, die wärmeempfindlich sind.

Nachfolgend wird in tabellarischer Form ein Ausführungsbeispiel der Erfindung wiedergegeben. Hierbei wurden 5 kg Backhefe behandelt.

1. Vorbereitung: - Auflösen der Backhefe in Wasser
- Auffüllen der Einschubbleche

2. Frosten im Gefrierschrank

| | Produktkerntemperatur | | Anlagentemperatur |
|---|---|---|---|
| | Anfang (°C) | Ende (°C) | (°C) |
| Einfrieren | +15 | -10 | -100 |
| Erwärmen | -10 | -2 | -2 |
| Tiefgefrieren | -2 | -60 | -100 |

Versuchsdauer: 4 h
(Durch das Erwärmen wird die Kristallbildung unterbrochen, so daß nur wenige große Kristalle im Zellinnern gebildet werden).

3. Vorzerkleinerung der Konzentratplatten auf einer Schneidmühle:
- Anlagentemperatur: - 60° C
- Konzentrat-Granulat: d = ca. 4 mm
- Schneiddauer: 0,1 h

4. Kaltmahlen über einen Wirbelschneckenkühler in einer Stiftmühle:
- Mühlentemperatur - 60° C
Produktkerntemperatur
- Anfang: - 60° C
- Ende: - 70° C
- Mahldauer: 0,25 h

Der hierbei erreichte Aufschlußgrad betrug 80 bis 90% und entspricht dabei völlig den Aufschlußgraden, die bei der Naßmahlung oder Hochdruckhomogenisation erreicht werden. Die Gesamtkosten sprechen annähernd denen der bekannten mechanischen Aufschlußverfahren der Naßmahlung und Hochdruckhomogenisation. Das erfindungsgemäße Verfahren besitzt demgegenüber jedoch die zusätzlichen genannten Vorteile.

## Ansprüche

1. Verfahren zum Aufschluß der Zellen von Mikroorganismen zwecks Gewinnung der Zellinhaltsstoffe, bei dem aus den Mikroorganismen ein Konzentrat gebildet wird,
dadurch gekennzeichnet,
daß das Konzentrat durch ein tiefsiedendes verflüssigtes Gas tiefgefroren und anschließend mechanisch aufgeschlossen wird.

2. Verfahren nach Anspruch 1,
dadurch gekennzeichnet,
daß das Konzentrat in Form von Platten tiefgefroren wird.

3. Verfahren nach Anspruch 1,
dadurch gekennzeichnet,
daß das Konzentrat durch Eintropfen in das tiefsiedende verflüssigte Gas zu Pellets tiefgefroren wird.

4. Verfahren nach einem der Ansprüche 1 bis 3,
dadurch gekennzeichnet,
daß das tiefgefrorene Konzentrat vor der Vermahlung längere Zeit zwischengelagert wird.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl⁵) |
|---|---|---|---|
| X | GB - A - 2 196 018 (FUJI SEIKI MACHINE WORKS LTD.) * Ansprüche 1,7-9 * -- | 1,3 | C 12 N 1/06 |
| A | AT - B - 305 (RÜCKFORTH) * Anspruch * ---- | 1 | |

| RECHERCHIERTE SACHGEBIETE (Int Cl⁵) |
|---|
| C 12 N |

Der vorliegende Recherchenbericht wurde für alle Patentansprüche erstellt.

| Recherchenort | Abschlußdatum der Recherche | Prüfer |
|---|---|---|
| WIEN | 13-06-1990 | WOLF |